(19) Europäisches Patentamt
European Patent Office
Office européen des brevets

(11) **EP 3 138 466 A1**

(12) **EUROPÄISCHE PATENTANMELDUNG**

(43) Veröffentlichungstag:
08.03.2017 Patentblatt 2017/10

(51) Int Cl.:
*A61B 1/005* (2006.01)    *G02B 23/24* (2006.01)
*A61B 17/295* (2006.01)    *A61M 25/01* (2006.01)
*A61B 1/00* (2006.01)

(21) Anmeldenummer: **16191510.3**

(22) Anmeldetag: **22.03.2012**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorität: **12.04.2011 DE 102011001973**

(62) Dokumentnummer(n) der früheren Anmeldung(en)
nach Art. 76 EPÜ:
**12710268.9 / 2 696 741**

(71) Anmelder: **Aesculap AG**
**78532 Tuttlingen (DE)**

(72) Erfinder:
• **Weißhaupt, Dieter**
**78194 Immendingen (DE)**

• **Breitling, Susanne**
**74722 Buchen (DE)**
• **Lutze, Theodor**
**78582 Balgheim (DE)**

(74) Vertreter: **Hoeger, Stellrecht & Partner**
**Patentanwälte mbB**
**Uhlandstrasse 14c**
**70182 Stuttgart (DE)**

Bemerkungen:
Diese Anmeldung ist am 29-09-2016 als
Teilanmeldung zu der unter INID-Code 62 erwähnten
Anmeldung eingereicht worden.

(54) **STEUERUNGSVORRICHTUNG**

(57) Es wird eine Steuerungsvorrichtung, insbesondere zur Verwendung in Endoskopen oder dergleichen, vorgeschlagen, mit der längere Zeit ermüdungsfrei gearbeitet werden kann. Die Steuerungsvorrichtung ist ausgestattet mit einem proximalen und einem distalen, jeweils eine Gelenkzone umfassenden Endabschnitt sowie einem dazwischen angeordneten Mittelabschnitt, mit einem äußeren hohlzylindrischen Schaft, einem inneren, vorzugsweise hohlzylindrischen Schaft sowie mit einem zwischen diesen Schäften angeordneten Steuerungselement mit zwei oder mehr sich im Wesentlichen vom proximalen zum distalen Endabschnitt der Steuerungsvorrichtung erstreckenden, Kraft übertragenden Längselementen zur mechanischen Kopplung der Bewegung des distalen Endabschnitts mit der Bewegung des proximalen Gelenkabschnitts, wobei die Längselemente in Umfangsrichtung der Steuerungsvorrichtung in im Wesentlichen regelmäßigen Winkelabständen $b_1$, $b_2$, ... $b_n$ voneinander beabstandet angeordnet und im Bereich ihrer proximalen und distalen Enden jeweils in Umfangsrichtung gegeneinander fixiert sind, wobei die Winkelabstände $b_1$, $b_2$, ... $b_n$ der Längselemente zueinander, in Umfangsrichtung gemessen, so gewählt sind, dass die Kraft übertragenden Längselemente in Umfangsrichtung berührungsfrei und gegebenenfalls in Radialrichtung an dem äußeren und/oder dem inneren Schaft anliegend geführt sind.

*FIG.3A*

**Beschreibung**

Die Erfindung betrifft eine Steuerungsvorrichtung für feinmechanische oder chirurgische Anwendungen, beispielsweise zur Verwendung in Endoskopen oder dergleichen.

Die Erfindung betrifft insbesondere eine Steuerungsvorrichtung für Instrumente für hochgenaue mechanische Anwendungen oder chirurgische Anwendungen im minimal-invasiven Bereich.

Solche Steuerungsvorrichtungen sind im Stand der Technik bekannt und weisen einen proximalen, das heißt dem Benutzer/Chirurgen zugewandten, und einen von ihm abgewandten oder distalen Endabschnitt auf, der jeweils eine Gelenkzone umfasst, sowie einen zwischen den Endabschnitten angeordneten, häufig biegesteif ausgestalteten Mittelabschnitt. Sie umfassen ferner einen äußeren hohlzylindrischen Schaft, einen inneren hohlzylindrischen Schaft sowie ein zwischen diesen Schäften angeordnetes Steuerungselement mit zwei oder mehr sich im Wesentlichen vom proximalen zum distalen Endabschnitt der Steuerungsvorrichtung erstreckenden, Kraft übertragenden Längselementen. Die Kraft übertragenden Längselemente werden in Umfangsrichtung der Steuerungsvorrichtung im Wesentlichen regelmäßig angeordnet und sind im Bereich des proximalen und des distalen Endabschnittes jeweils in Umfangsrichtung miteinander verbunden. Über die Längselemente lassen sich Zug- und vorzugsweise auch Druckkräfte übertragen, mit denen sich eine Schwenkbewegung des proximalen Endabschnitts in eine hierzu korrelierende Schwenkbewegung des distalen Endabschnitts umsetzen lässt.

Am proximalen Ende der eingangs beschriebenen Steuerungsvorrichtung wird in der Regel ein von Hand zu betätigendes Griffteil montiert, an dessen Stelle selbstverständlich auch Motor-betriebene Bedienelemente treten können, während an dem distalen Ende, das auch Kopf genannt wird, Werkzeuge, Kameras, Beleuchtungselemente und dergleichen angeschlossen werden können.

Mit solchen die Steuerungsvorrichtung beinhaltenden Instrumenten lassen sich im Bereich der Mechanik beispielsweise komplizierte und schwer zugängliche Innenräume, beispielsweise von Motoren, Maschinen, Radiatoren und dergleichen, inspizieren und reparieren oder aber im Bereich der Chirurgie die oben angesprochenen Operationen in minimal-invasiver Technik durchführen.

Steuerungsvorrichtungen der eingangs beschriebenen Art sind z.B. aus der WO 2005/067785 A1 bekannt, bei der eine Vielzahl von Kraft übertragenden Längselementen in Form von Drähten oder Kabeln verwendet werden, welche in Umfangsrichtung direkt aneinander anliegend angeordnet sind und sich so gegenseitig seitlich führen. Für die Führung der Kraft übertragenden Längselemente in Radialrichtung stehen der äußere und der innere hohlzylindrische Schaft zur Verfügung, so dass eine Führung der Kraft übertragenden Längselemente in jeder Richtung gewährleistet ist.

In ähnlicher Weise arbeiten auch die in der WO 2009/098244 A2 offenbarten Steuerungsvorrichtungen, bei denen anstelle der Kabel oder Drähte ein geschlitztes Röhrchen als Steuerungselement verwendet wird. Das Steuerungselement umfasst eine Vielzahl durch Schlitze im Röhrchen definierter Längselemente, die durch ungeschlitzte Endbereiche des Röhrchens zusammengehalten werden.

Für die Funktion des in der WO 2009/098244 A2 beschriebenen Steuerungselements ist es wichtig, dass die Längselemente in den Gelenkbereichen der Steuerungsvorrichtung drahtförmig und damit flexibel und im zwischen den Gelenkbereichen liegenden Mittelabschnitt biegefest ausgebildet sind. Während ein Steuerungselement aus einem Röhrchen gefertigt für den Zusammenbau der Steuerungsvorrichtung gewisse Vorteile bietet, ist andererseits die Fertigung des geschlitzten Röhrchens aufwändig, insbesondere auch weil der Mittelabschnitt der Längselemente biegesteif sein muss.

In letzterer Hinsicht ist das Konzept der WO 2009/112060 günstiger, bei dem das Erfordernis der biegesteifen Mittelabschnitte der Längselemente entfällt. Allerdings erfordert die Funktion der Kraftübertragung durch die Längselemente auch hier eine gegenseitige seitliche Führung, die mit dem Auftreten von weiteren Reibungskräften verbunden ist.

Nachteilig bei den bisher bekannten Steuerungsvorrichtungen ist, dass aufgrund des Bauprinzips bei einer Betätigung Reibungskräfte zwischen den Längselementen wirken, die zumindest bei länger andauerndem Gebrauch für den Benutzer, z.B. den Chirurgen, ermüdend wirken. Dies ist insbesondere deshalb nachteilig, weil es für den Patienten ein Risiko bedeutet.

Aufgabe der Erfindung ist es, eine Steuerungsvorrichtung vorzuschlagen, mit der längere Zeit ermüdungsfrei gearbeitet werden kann.

In diesem Zusammenhang schlägt die Erfindung vor, dass die erfindungsgemäße Steuerungsvorrichtung einen proximalen und einen distalen, jeweils eine Gelenkzone umfassenden Endabschnitt sowie einen dazwischen angeordneten Mittelabschnitt aufweist und einen äußeren hohlzylindrischen Schaft, einen inneren, vorzugweise hohlzylindrischen Schaft sowie ein zwischen diesen Schäften angeordnetes Steuerungselement mit zwei oder mehr, sich im Wesentlichen vom proximalen zum distalen Endabschnitt der Steuerungsvorrichtung erstreckenden, Kraft übertragenden Längselementen zur mechanischen Kopplung der Bewegung des distalen Endabschnitts mit der Bewegung des proximalen Gelenkabschnitts umfasst, wobei die Längselemente in Umfangsrichtung der Steuerungsvorrichtung in im Wesentlichen regelmäßigen Winkelabständen $b_1$, $b_2$, ... $b_n$ voneinander beabstandet angeordnet und im Bereich ihrer proximalen und distalen Enden jeweils in Umfangsrichtung gegeneinander fixiert sind und wobei die Winkelabstände $b_1$, $b_2$, ... $b_n$ der

Längselemente zueinander in Umfangsrichtung gemessen so gewählt sind, dass die Kraft übertragenden Längselemente in Umfangsrichtung berührungsfrei und gegebenenfalls in Radialrichtung an dem äußeren und/oder dem inneren Schaft anliegend geführt sind.

Die erfindungsgemäße Steuerungsvorrichtung ermöglicht damit ein ermüdungsfreies Arbeiten aufgrund der gegenüber herkömmlichen Steuerungsvorrichtungen wesentlich verringerten, auf die Längselemente wirkenden Reibungskräften. Außerdem wird das Risiko des Materialabriebs vermindert.

Es ist gemäß der vorliegenden Erfindung möglich, die Führung der Längselemente auf einen Kontakt mit dem äußeren und/oder dem inneren Schaft zu beschränken und so weiterhin die Reibungskräfte zu reduzieren.

Die Längselemente können dabei sowohl in den Gelenkbereichen als auch im Mittelabschnitt eine im Wesentlichen gleiche Flexibilität aufweisen, insbesondere auch im Wesentlichen über ihre gesamte Länge gleich flexibel sein. Damit lassen sich kostengünstig hergestellte Längselemente verwenden.

Für Steuerungsvorrichtungen, die im Betrieb größere Kräfte übertragen müssen, ist allerdings bevorzugt, wenn die Längselemente in Radialrichtung an dem äußeren und dem inneren Schaft anliegend geführt sind.

Dies bietet die Sicherheit, dass ein exakter Bewegungsablauf auch dann gewährleistet ist, wenn die Kräfte, die auf die Längselemente wirken, eine geringe elastische Verformung der Längselemente hervorrufen könnten.

Werden zwei Kraft übertragende Längselemente verwendet, ist die Schwenkbewegung auf eine Ebene beschränkt. Werden mehrere, insbesondere vier Kraft übertragende Längselemente verwendet, besteht die Möglichkeit, die Steuerungsvorrichtung in zwei aufeinander senkrecht stehenden Ebenen zu verschwenken oder aber, insbesondere bei der Verwendung von sechs Steuerungselementen oder mehr, beispielsweise acht, in praktisch beliebig wählbaren Ebenen zu verschwenken.

Der Querschnitt der Längselemente wird vorzugsweise im Wesentlichen als Kreisbogensegment oder weiter bevorzugt im Wesentlichen rechteckförmig ausgebildet.

Während die Form des Kreisbogensegments eine Verbesserung der Quersteifigkeit der Längselemente schafft, liegt die Vorteile des im Wesentlichen rechteckigen Querschnitts der Längselemente zum einen in einer gut ausgeprägten Quersteifigkeit und zum anderen in einer Minimierung der Kontaktflächen der Längselemente gegenüber dem oder den eine Führung bietenden äußeren und/oder inneren Schaft oder Schäften.

Für die Herstellung der erfindungsgemäß zu verwendenden Längselemente bzw. des daraus gebildeten Steuerungselements gibt es mehrere bevorzugte Möglichkeiten.

Zunächst kann das Steuerungselement mit seinen Längselementen von einem längs geschlitzten Rohr gebildet werden, wobei die Endbereiche des Rohrs vorzugsweise ungeschlitzt bleiben und so die Längselemente in Umfangsrichtung gegeneinander fixieren. Die Schlitze werden vorzugsweise in radialer Richtung in die Wandung des Rohrs, beispielsweise durch Laserschneiden eingebracht. Je nach Elastizität des Rohrmaterials und verwendeter Schneidetechnik können die entstehenden Schnittspalte so dimensioniert werden, dass damit bereits ausreichende Winkelabstände zwischen den Längselementen erhalten werden, um diese auch bei Krafteinwirkung berührungsfrei zu halten. Die Längselemente weisen hier einen kreisbogenförmigen Querschnitt auf.

Fertigungstechnisch einfacher ist die Herstellung des erfindungsgemäß zu verwendenden Steuerungselements, wenn man ein längs geschlitztes, erst nachfolgend zum Rohr geformtes Flachmaterial verwendet. Neben dem erwähnten Fertigungsvorteil ergibt sich des Weiteren der Vorteil, dass die Auswahl an Rohmaterialen erheblich größer ist und diese zu günstigeren Preisen bezogen werden können. Ferner sind die Flachmaterialien mit geringeren Toleranzen herstellbar als die zuerst erwähnten Rohre.

Schließlich weisen die Längselemente, die aus dem Flachmaterial hergestellt sind, statt des kreisbogenförmigen Querschnitts einen im Wesentlichen rechteckigen Querschnitt auf, bei dem die Kontaktflächen mit den Führung gebenden Schäften und damit die Reibungskräfte beim Betrieb der Steuerungsvorrichtung, wie schon erwähnt, weiter erheblich vermindert sind.

Die Endbereiche des Flachmaterials in Axialrichtung können ungeschlitzt bleiben, so dass die Längselemente in Umfangsrichtung bereits gegeneinander fixiert sind. Auch hier können je nach Material und Fertigungstechnik die bei der Herstellung der Schlitze entstehenden Schnittspalte zwischen den Längselementen ausreichende Winkelabstände im fertigen Steuerungselement schaffen.

Alternativ werden die Längselemente bevorzugt zunächst als einzelne stabförmige Elemente aus einem Flachmaterial gefertigt, welche an ihren proximalen und distalen Enden, beispielsweise unter Verwendung eines Ringbunds, in Umfangsrichtung gegeneinander festgelegt werden. Hier ergeben sich dieselben Vorteile wie bei dem aus einem Flachmaterial hergestellten Steuerungselement wie oben beschreiben. Zusätzlich ergibt sich der Vorteil, dass bei der Fixierung der Längselemente in Umfangsrichtung gegeneinander verschiedene, auf die jeweilige Applikation der Steuerungsvorrichtung angepasste Techniken verwendet werden können, die im Zusammenhang mit der Beschreibung der Figuren noch näher erläutert werden. Wird ein Ringbund verwendet, kann dieser auch in komplexer Form für einen Formschluss mit den Enden der Längselemente, beispielsweise durch Erodieren oder mittels Powder Injection Molding (PIM), hergestellt werden. Bevorzugt sind auch Ringbünde, die aus Oxidkeramik hergestellt sind.

Das Steuerungselement braucht nicht zwingend am inneren und äußeren Schaft anzuliegen. Vielmehr wird bevorzugt ein Spalt auf mindestens der Innen- oder der Außenseite des Steuerungselements gegenüber der Außenoberfläche des inneren Schafts bzw. der Innenoberfläche des äußeren Schafts vorgesehen, welcher in der Ruhestellung des Steuerungselements vorzugsweise ca. 0,01 bis 0,5 mm beträgt.

Zur weiteren Optimierung des Gleitverhaltens gegeneinander können die Innenoberfläche des äußeren Schafts, die Längselemente und/oder die Außenoberflächen des inneren Schafts mit einem die Gleitreibung herabsetzenden Material, z.B. PTFE, beschichtet sein.

Bei einer bevorzugten Steuerungsvorrichtung liegt der Außendurchmesser des äußeren Schafts im Bereich von ca. 1 mm bis ca. 20 mm und der Innendurchmesser des inneren Schafts im Bereich von ca. 0,05 mm bis ca. 5 mm, insbesondere ca. 0,2 mm bis ca. 5 mm.

Diese Abmessungen sind in vielen Fällen typisch für in der Endoskopie einzusetzende Steuerungsvorrichtungen und erlauben die Verwendung von Instrumenten und Werkzeugen, die am distalen Ende der Steuerungsvorrichtung angeordnet und über das Lumen des inneren Schafts angesteuert und betätigt werden können.

Es versteht sich, dass für andere Anwendungen selbstverständlich Steuerungsvorrichtungen mit von den vorstehend genannten Bereichen erheblich abweichenden Abmessungen denkbar sind.

Die Längselemente des Steuerungselements können in vielen Fällen geradlinig ausgebildet sein. Eine Bewegung des proximalen Endabschnitts wird dann in eine entsprechende Verschwenkbewegung des distalen Endabschnitts umgesetzt, die in derselben Ebene wie die des proximalen Endes und in gleicher Schwenkrichtung erfolgt.

Während dieser Bewegungsablauf in einer Vielzahl von Applikationen ausreichenden Zugang zu den jeweiligen Einsatzorten der an der Steuerungsvorrichtung angeordneten Instrumente und Werkzeuge bietet, gibt es Applikationen, in denen eine anders geartete Umsetzung der Bewegungen des proximalen und distalen Endabschnitts wünschenswert ist.

Hierzu schlägt die vorliegende Erfindung vor, dass die distalen Enden der Längselemente in Umfangsrichtung in Winkelpositionen festgelegt sind, die von den Winkelpositionen, in denen die jeweils zugehörenden proximalen Enden festgelegt sind, verschieden sind, wobei die Winkelpositionen vorzugsweise in Umfangsrichtung um ca. 10 ° bis 350 °, insbesondere um ca. 45 ° bis 315 °, weiter bevorzugt im Bereich von ca. 150 ° bis ca. 210 ° differieren. Die Schwenkbewegungen des distalen und proximalen Endabschnitts finden dann nicht mehr in derselben Ebene und/oder derselben Schwenkrichtung statt.

Besondere Bedeutung haben Steuerungsvorrichtungen der vorliegenden Erfindung, bei denen die Winkelpositionen eine Differenz von ca. 180 ° aufweisen, so dass eine spiegelbildliche Bewegung des proximalen und distalen Endabschnittes in einer Ebene erzeugt werden kann.

Die Anordnung der Längselemente in Umfangsrichtung zur Erzielung der unterschiedlichen Winkelpositionen am proximalen und distalen Ende kann in verschiedener Weise erreicht werden.

Im Zusammenhang mit einer solchen Ausführungsform kann insbesondere vorgesehen sein, dass die Kraft übertragenden Längselemente mindestens über einen Teil ihrer Länge schraubenlinienförmig zwischen den Schäften angeordnet sind.

Bei einer bevorzugten Ausführungsform sind die Kraft übertragenden Längselemente über ihre gesamte Länge schraubenlinienförmig zwischen den Schäften angeordnet. Hier ergibt sich im Hinblick auf die typische Länge der Steuerungsvorrichtung von 10 cm und deutlich mehr und bei einem typischen Durchmesser von wenigen Millimetern eine extrem hohe Steigung der Schraubenlinienform oder, anders ausgedrückt, eine sehr geringe Abweichung von der Parallelität zur Längsrichtung der Steuerungsvorrichtung, die wenige Winkelgrade, beispielsweise ca. 5 ° oder weniger, ausmacht.

Gemäß einer Variante dieser Ausführungsform sind die Kraft übertragenden Längselemente im Bereich der proximalen und/oder distalen Enden im Wesentlichen parallel zur Längsrichtung der Steuervorrichtung und in einem dazwischen liegenden Bereich schraubenlinienförmig angeordnet.

Gemäß einer weiteren Variante weisen die Kraft übertragenden Längselemente einen oder mehr Abschnitte im Bereich zwischen ihren proximalen und distalen Enden auf, welche parallel zur Längsrichtung der Steuerungsvorrichtung angeordnet sind. Andere Abschnitte, insbesondere benachbart zum proximalen und distalen Ende sind schraubenlinienförmig angeordnet.

Obwohl bei den letzten beiden Varianten nur ein Teil der Gesamtlänge des Steuerungselements zur Erzielung des Winkelversatzes zur Verfügung steht, werden immer noch nur geringe Winkelabweichungen von der Längsrichtung notwendig, z.B. ca. 5° oder weniger.

Für die Ausbildung der Gelenkabschnitte bieten sich vielfältige Möglichkeiten an.

Im Stand der Technik wurden vielfältig in Umfangsrichtung geschlitzte Schaftabschnitte hierfür vorgeschlagen, so z.B. in der US 5,741,429.

Ähnliche Vorschläge für die Ausbildung der Gelenkabschnitte sind auch der WO 2009/098244 A2 und der WO 2009/112060 A1 zu entnehmen.

Hier können die Gelenkzone(n) des äußeren und/oder inneren Schafts einen Wandabschnitt umfassen, in dem mehrere voneinander beabstandete, in Umfangsrichtung verlaufende Schlitze angeordnet sind, welche bevorzugt als die Zylinderwand des Schafts vollständig durchdringende Schlitze ausgebildet sind, wobei vorzugsweise in Umfangsrichtung zwei oder mehr, insbesondere drei oder mehr Schlitze hintereinander angeordnet sind.

Bevorzugte Gelenkzonen dieser Ausführungsform weisen in Axialrichtung drei oder mehr Schlitze nebeneinander und optional gegeneinander versetzt angeordnet auf.

Hierbei kann vorgesehen sein, dass mindestens eine der Gelenkzonen elastisch ausgebildet ist, so dass ein vom Steuerungselement ausgeübter Rückstelleffekt verstärkt wird.

Während diese an sich bekannten Ausführungsformen der Gelenkabschnitte sich für die erfindungsgemäße Steuerungsvorrichtung durchaus eignen, werden die Gelenkabschnitte bei alternativen Ausführungsformen so gestaltet, dass diese beim inneren und/ oder äußeren Schaft eine Mehrzahl an separaten Ringsegmenten umfassen, welche in Axialrichtung jeweils einen ersten und einen zweiten Endbereich aufweisen, wobei der erste Endbereich zwei oder mehr in Axialrichtung abstehende Vorsprünge und der zweite Endbereich zwei oder mehr die Vorsprünge aufnehmende Rücksprünge umfasst, wobei die Vor- und Rücksprünge in regelmäßigen Abständen in Umfangsrichtung am jeweiligen Ringsegment angeordnet sind, wobei die Ringsegmente über die Vor- und Rücksprünge gelenkig ineinander greifen und wobei die Ringsegmente über die Vor- und Rücksprünge in Axial- und/oder Radialrichtung miteinander formschlüssig verbunden sind.

Diese Art der Gelenkabschnitte vermindert den Kraftaufwand bei der Betätigung der Steuerungsvorrichtung wiederum erheblich.

Das erfindungsgemäß ausgebildete Steuerungselement kann mit elastisch aus der Axialrichtung auslenkbaren Längselementen ausgebildet werden, welche eine Rückstellkraft in den Gelenkabschnitten ausüben, die für die Steuerungsvorrichtung als Ganzes ausreichend ist. Die Gelenkabschnitte des äußeren und inneren Schafts müssen keinen Beitrag zur Rückstellfunktion leisten. Der Formschluss der benachbarten Ringsegmente ist dabei ein lockeres Ineinandergreifen von Vor- und Rücksprüngen.

Die Vor- und Rücksprünge sind typischerweise in Umfangsrichtung der Schäfte in regelmäßigen Abständen angeordnet.

Die Wahl einer ungeraden Anzahl von Vor- und Rücksprüngen stellt sicher, dass die Gelenkabschnitte bzw. deren Ringsegmente mechanisch gesichert sind und die einzelnen Segmente unverlierbar integriert sind. Dies ermöglicht einen vereinfachten Zusammenbau der Steuerungsvorrichtung.

Ergänzend oder alternativ kann vorgesehen sein, dass die Rücksprünge auf der radial innen liegenden Seite in Umfangsrichtung eine Ausdehnung aufweisen, die kleiner ist als die entsprechende radial außen liegende Ausdehnung des in den Rücksprung eingreifenden Vorsprungs.

Weiter bevorzugte Steuerungsvorrichtungen beinhalten, dass die Vorsprünge an ihrem dem Ringsegment abgewandten freien Ende in Umfangsrichtung eine größere Ausdehnung aufweisen als benachbart zum Ringsegment und dass die Rücksprünge an ihrem offenen Ende eine kleinere Ausdehnung in Umfangsrichtung aufweisen als die Ausdehnung des in den Rücksprung eingreifenden Vorsprungs an seinem freien Ende.

Mit dieser Maßnahme allein kann schon eine gegenseitige Sicherung der Ringsegmente vorgenommen werden.

Weiter bevorzugt sind die Vor- und die Rücksprünge in Umfangs- und/oder Radialrichtung mit einem im Wesentlichen trapezförmigen Querschnitt ausgebildet.

Bevorzugte Materialien für die Herstellung der Längselemente des Steuerungselements umfassen Stahllegierungen und Nitinol.

Für einige Applikationen kann die Steuerungsvorrichtung über ihre gesamte Länge flexibel ausgestaltet sein, so dass beim Einführen in das Operationsfeld ein gerader Zugangskanal nicht zwingend ist. Für hochpräzise Aufgabenstellungen ist es jedoch häufig wünschenswert, wenn mindestens einer der äußeren und inneren Schäfte einen zwischen den proximalen und distalen Gelenkzonen angeordneten biegesteifen Abschnitt aufweist.

Typischerweise ist die Steuerungsvorrichtung in ihrem Mittelabschnitt biegesteif ausgebildet.

Gemäß einer Ausführungsform der Erfindung ist mindestens einer der äußeren und inneren Schäfte im Bereich zwischen den proximalen und distalen Gelenkzonen mit einem biegesteifen Abschnitt ausgerüstet, der die Biegesteifigkeit des Mittelabschnittes der Steuerungsvorrichtung realisiert.

Während in vielen Fällen die proximale und die distale Gelenkzone gleich ausgebildet sind und insbesondere eine gleiche Ausdehnung in Längsrichtung der Steuerungsvorrichtung aufweisen, ist dies nicht zwingend erforderlich.

Insbesondere kann vorgesehen sein, dass die proximale und die distale Gelenkzone verschieden, insbesondere auch verschieden lang, ausgebildet sind, so dass sich eine entsprechende Schwenkbewegung der proximalen Gelenkzone in einer geringeren oder verstärkten Schwenkbewegung des distalen Endabschnitts auswirkt.

Insbesondere kann vorgesehen sein, dass die Schwenkbewegung der proximalen und/oder distalen Gelenkzone einstellbar ist. Dies kann beispielsweise geschehen, indem die Ausdehnung der proximalen und/oder der distalen Gelenkzone variiert wird und damit das Schwenkverhalten der beiden Gelenkzonen zueinander verändert wird.

Insbesondere kann vorgesehen sein, dass die Steuerungsvorrichtung eine Haltevorrichtung umfasst, mit der Teile einer der Gelenkzonen biegefest bezüglich des Mittelabschnittes der Steuerungsvorrichtung oder einer sich an deren proximalen oder distalen Endabschnitt anschließenden Funktionseinheit fixierbar sind.

So kann bei einer Variante der erfindungsgemäßen Steuerungsvorrichtung die Haltevorrichtung eine parallel zur Längsachse des Mittelabschnittes, der in diesem Fall biegesteif ausgebildet ist, verschiebliche, biegesteife Hülse umfassen.

Je nach Position der Hülse in Längsrichtung zum Mittelabschnitt kann der proximale und/oder distale Endabschnitt und die dort vorgesehene Gelenkzone in ihrer Länge beeinflusst und damit auch in ihrem Schwenkverhalten beeinflusst werden.

Vorzugsweise wird dabei die biegesteife Hülse am Außenumfang des biegesteifen Schafts angeordnet, so dass das Lumen der Steuerungsvorrichtung unbeeinflusst bleibt. Sollte das Lumen der Steuerungsvorrichtung für bestimmte Anwendungsfälle ausreichend groß sein, kann natürlich eine biegesteife Hülse auch im Inneren des Lumens angeordnet werden. Einfacher sind jedoch die Verschieblichkeit und insbesondere auch die Festlegung der biegesteifen Hülse zu realisieren, wenn diese am Außenumfang des äußeren Schaftes angeordnet ist.

Gemäß einer anderen Variante kann die Haltevorrichtung an der Funktionseinheit, die an das proximale oder distale Ende der Steuerungsvorrichtung gekoppelt ist, ein abstützendes Halteelement umfassen. Auf diese Weise lässt sich die Gelenkzone von Seiten des distalen bzw. proximalen Endes her in ihrem Schwenkverhalten beeinflussen.

Gemäß einer weiteren Variante der erfindungsgemäßen Steuerungsvorrichtung ist die Haltevorrichtung in einer vorgegebenen Stellung positionierbar und insbesondere auch festlegbar. Damit besteht die Möglichkeit, wiederholbar und genau vorgebbar das Schwenkverhalten von distalem und proximalem Endabschnitt zueinander vorab einzustellen oder wieder einzustellen.

Gemäß einer weiteren Variante der erfindungsgemäßen Steuerungsvorrichtung ist vorgesehen, dass mindestens eine der Gelenkzonen elastisch ausgebildet ist, so dass, wenn die zur Verschwenkung der Endabschnitte eingeleiteten Kräfte aufhören zu wirken, sich die Steuerungsvorrichtung wieder in ihre ursprüngliche gerade Stellung zurückstellt.

Diese und weiter Vorteile der Erfindung werden im Folgenden anhand der Zeichnung noch näher erläutert. Es zeigen im Einzelnen:

Figur 1        eine perspektivische Darstellung eines chirurgischen Instruments mit einer erfindungsgemäßen Steuerungsvorrichtung;

Figur 2        eine Explosionsdarstellung der Steuerungsvorrichtung der Figur 1;

Figur 2A        ein Detail einer Gelenkzone der Vorrichtung der Figur 2;

Figuren 3A und B        Details von Teilen der Steuerungsvorrichtung der Figur 2;

Figuren 4A und B        Schnittdarstellung entlang Linie IV - IV in Figur 1 und eine alternativen Ausführungsform hiervon;

Figuren 5A bis C        Details einer alternativen erfindungsgemäßen Steuerungsvorrichtung;

Figur 5D        einen Querschnitt, längs Linie VD-VD von Figur 5A ergänzt um einen äußeren Schaft;

Figuren 6A bis E        verschiedene alternative Ausführungsformen von Längselementen zur Bildung eines Steuerungselements einer erfindungsgemäßen Steuerungsvorrichtung;

Figuren 7A bis C        verschiedene alternative Ausführungsformen von Längselementen zur Bildung eines Steuerungselements einer erfindungsgemäßen Steuerungsvorrichtung; und

Figur 8        Variante eines Gelenkabschnitts eines Schaftes einer erfindungsgemäßen Steuerungsvorrichtung.

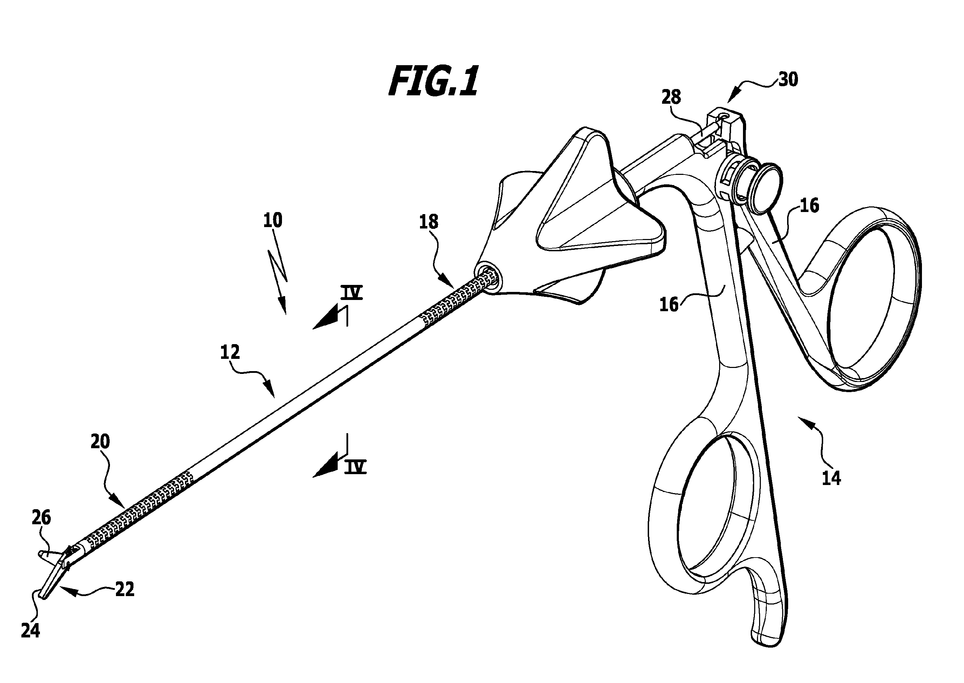

Figur 1 zeigt ein insgesamt mit dem Bezugszeichen 10 bezeichnetes chirurgisches Instrument mit einer erfindungsgemäße Steuerungsvorrichtung 12 und einem Handgriff 14 mit zwei gegeneinander verschwenkbaren Branchen 16, der lösbar mit einem proximalen Ende 18 der Steuerungsvorrichtung 12 verbunden ist.

Am distalen Ende 20 der Steuerungsvorrichtung 12 ist ein Schneidwerkzeug 22 fixiert, dessen Scherblätter 24, 26 gegeneinander verschwenkbar gelagert sind. Die Verschwenkbewegung der Scherblätter 24, 26 wird über die Verschwenkung der Branchen 16 bewirkt, wobei Verschwenkbewegungen der Branchen 16 über ein innerhalb der Steuerungsvorrichtung 12 geführtes, zylindrisches Kopplungselement 28 auf die Scherblätter 24, 26 des Schneidwerkzeugs 22 übertragen werden. Das Kopplungselement 28 ist proximal mit einer der Branchen lösbar in einem Rastsitz 30 verbunden, während sein distales Ende (nicht gezeigt) mit einem Schwenkmechanismus (nicht gezeigt) des Schneidwerkzeugs 22 in an sich bekannter Weise verbunden ist.

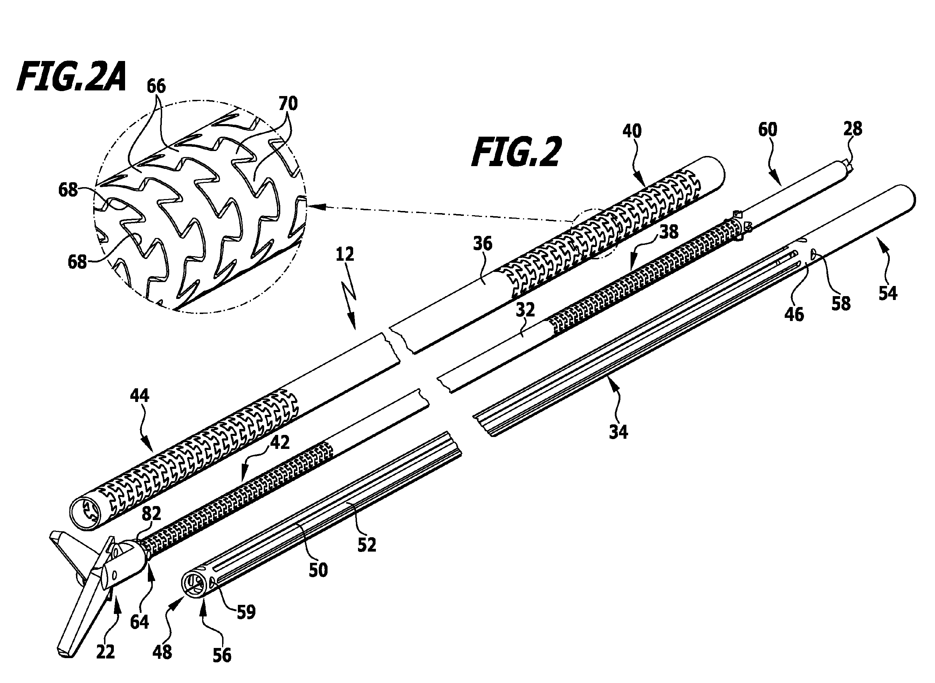

Figur 2 zeigt die Steuerungsvorrichtung 12 im Detail. Die Steuerungsvorrichtung 12 umfasst einen inneren Schaft 32, ein Steuerungselement 34 sowie einen äußeren Schaft 36. Im Innern des inneren Schafts ist das Kopplungselement 28 geführt.

Die inneren und äußeren Schäfte 32, 36 sind jeweils mit proximalen und distalen Gelenkzonen 38, 40 bzw. 42, 44 ausgebildet.

Das Steuerungselement 34 ist in der hier gezeigten Ausführungsform aus einem zum Rohr geformten, streifenförmigen Flachmaterial 46 gebildet dessen laterale Enden in dem mit dem Bezugszeichen 48 bezeichneten Bereich zusammengeführt sind. Das Zusammenführen kann lose geschehen oder mittels einer Naht 48 gesichert werden, beispielsweise mittels Schweißen oder Kleben.

Das streifenförmige Flachmaterial 46 wird der Länge nach mit Schlitzen 50 versehen, die einen Abstand zwischen Längselementen 52 definieren. Die Schlitze 50 verlaufen zwischen einem proximalen Endbereich 54 des Flachmaterials und seinem distalen Endbereich 56.

Die Endbereiche 54, 56 bleiben ungeschlitzt und verbinden damit die Längselemente 52 an ihren proximalen und distalen Enden in Form eines Ringbundes.

Die Schlitze erstrecken sich bis in die Gelenkbereiche der inneren und äußeren Schäfte hinein und gegebenenfalls auch über diese hinaus. So ist sichergestellt, dass ohne weitere Maßnahmen die Gelenkfunktion der Gelenkabschnitte von dem Steuerungselement 34 unterstützt wird.

Der proximale Endbereich 54 ist mit Bohrungen 58 versehen, die eine mechanische Verbindung mit dem proximalen Endbereich 60 des inneren Schafts 32 erlauben. Am distalen Endbereich 56 sind ebenfalls Bohrungen 59 vorgesehen, die eine mechanische Verbindung des distalen Endbereichs 56 Steuerungselements 34 mit dem distalen Endbereich 64 des inneren Schaft 32 ermöglichen.

Die mechanische Verbindung kann über einen bloßen Formschluß oder auch mittels einer stoffschlüssigen Verbindung, beispielsweise durch Kleben oder Schweißen, erfolgen.

Die proximalen und distalen Gelenkabschnitte 38, 40 bzw. 42, 44 des inneren und äußeren Schafts 32, 36 können gleichartig gestaltet werden.

Im vorliegenden Ausführungsbeispiel sind die Gelenkabschnitte 38, 40, 42, 44 jeweils aus mehreren Ringsegmenten 66 gebildet, wie dies in der Vergrößerung der Figur 2A ersichtlich ist. Die Ringsegmente 66 weisen an einem axialen Ende regelmäßig über den Umfang verteilt Rücksprünge 68 auf, während am anderen axialen Ende Vorsprünge 70 ausgebildet sind. Die Rücksprünge 68 und Vorsprünge 70 sind komplementär ausgebildet, so dass sich ein Formschluß ergibt. Vorzugsweise werden der innere und der äußere Schaft aus einstückigen Rohren gefertigt, wobei die Gelenkabschnitte beispielsweise durch Laserschneiden erzeugt werden. Wählt man die Anzahl der Vor- und Rücksprünge ungerade, beispielsweise 7, so ist sichergestellt, dass die Ringsegmente nach der Fertigung auch bei einem losen ineinander greifen unverlierbar miteinander verbunden bleiben.

Mittels einer Trapezform der Vor- und Rücksprünge 68 bzw. 70 in Umfangs- und vorzugsweise auch in radialer Richtung kann auch bei Verwendung einer geraden Anzahl an Vor- und Rücksprüngen eine unverlierbare, und trotzdem gelenkige Verbindung zwischen benachbarten Ringsegmenten 66 hergestellt werden.

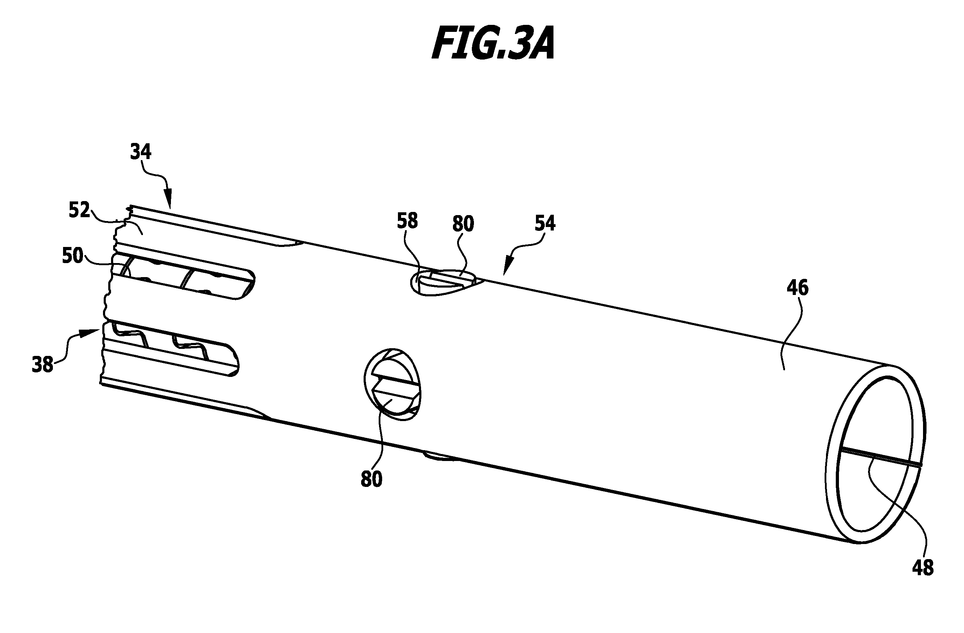

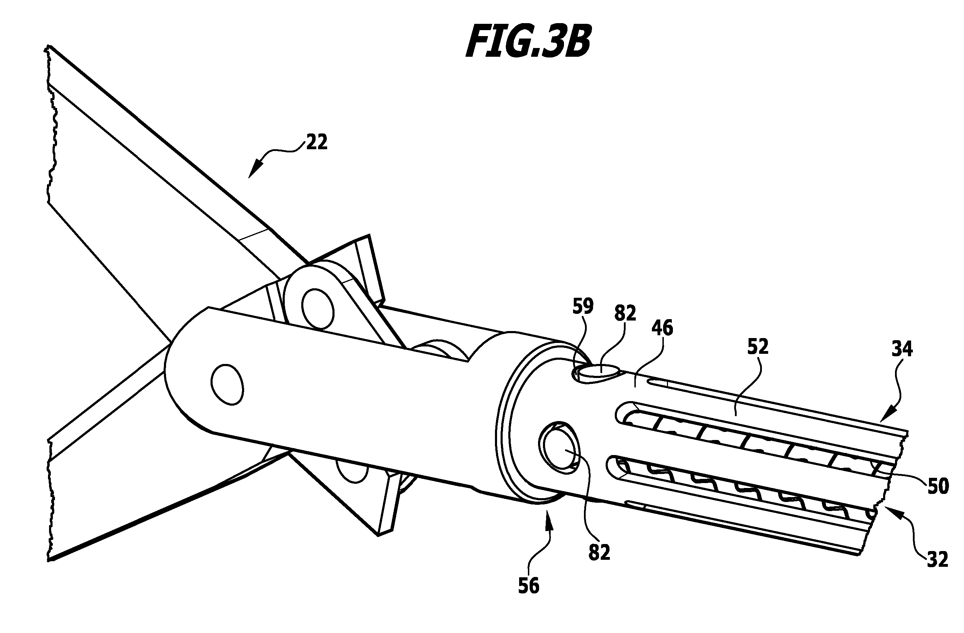

Die Figuren 3A und 3B zeigen noch einmal Details der erfindungsgemäßen Steuerungsvorrichtung 12 am proximalen bzw. distalen Endbereich.

Figur 3A zeigt das Steuerungselement 34 mit dem zum Rohr geformten Flachmaterial 46, welches sechs über Schlitze 50 getrennte Längselemente 52 sowie den ungeschlitzten Bereich 54 umfasst, in dem in Umfangsrichtung im Wesentlichen gleichmäßig verteilt Durchgangsöffnungen 58 vorgesehen sind, welche Vorsprünge aufnehmen, die am proximalen Endbereich 60 des inneren Schafts 32 radial nach außenabstehend vorgesehen sind. Wie in Figur 3A im Einzelnen dargestellt, können die Vorsprünge als Schraubenköpfe 80 ausgebildet sein.

Im Inneren des Steuerungselements 34 ist der Gelenkabschnitt 38 des inneren Schafts 32 zu sehen, der aus Ringsegmenten 66 wie oben beschrieben gebildet ist.

Figur 3B zeigt im Einzelnen die Ausbildung des distalen Endbereichs 56 des Steuerungselements 34, wobei wiederum der Endbereich 56 mit Durchgangsöffnungen 59 versehen ist, die zapfenförmige Vorsprünge 82, die am distalen Endbereich 64 des inneren Schafts 32 ausgebildet sind, aufnehmen, wenn das Flachmaterial 46, aus dem das Steuerungselement 34 gebildet ist, zum Rohr geformt wird. Anstelle der zapfenförmigen Vorsprünge könnten auch wie in Figur 3A für den proximalen Endbereich gezeigt Schraubenköpfe treten und umgekehrt.

An den distalen Endbereich 56 des Steuerungselements 34 schließt sich dann, wie zuvor beschrieben, das Schneidwerkzeug 22 an, auf dessen nähere Ausgestaltung, die an sich bekannt ist, hier nicht eingegangen zu werden braucht.

In der Figur 3B ist auch die Ausbildung des distalen Gelenkbereichs 42 des inneren Schafts 32 ersichtlich, wobei erneut Ringsegmente 66 wie oben beschrieben miteinander gelenkig in Eingriff stehend Verwendung finden.

Bei dem in den Figuren 1 bis 3 gezeigten chirurgischen Instrument ist die Steuerungsvorrichtung 12 so ausgebildet, dass aufgrund der Elastizität der Längselemente 52, die aus dem Flachmaterial 46 gebildet wurden, eine ausreichende Rückstellkraft zur Verfügung gestellt wird, so dass im unbelasteten Zustand das chirurgische Instrument bzw. dessen Steuerungsvorrichtung 12 eine gerade Konfiguration aufweist.

Wird am proximalen Ende 18 der Steuerungsvorrichtung 12 der Handgriff 14 gegenüber der Längsachse der Steuerungsvorrichtung 12 in Figur 1 beispielsweise nach unten ausgelenkt, wird gleichzeitig und in gleichem Maße das distale Ende 20 der Steuerungsvorrichtung nach oben verschwenkt. Wird die Kraft auf den Handgriff 14 wieder gelöst, stellt sich die Steuerungsvorrichtung 12 selbständig in die gerade Konfiguration zurück.

Für den Rückstelleffekt sind, wie erwähnt, die Elastizität der Längselemente 52 verantwortlich, während die aus Ringsegmenten 66 gebildeten Gelenkabschnitte 38, 40, 42, 44 der inneren und äußeren Schäfte 32 und 36 keinen Beitrag zu leisten brauchen.

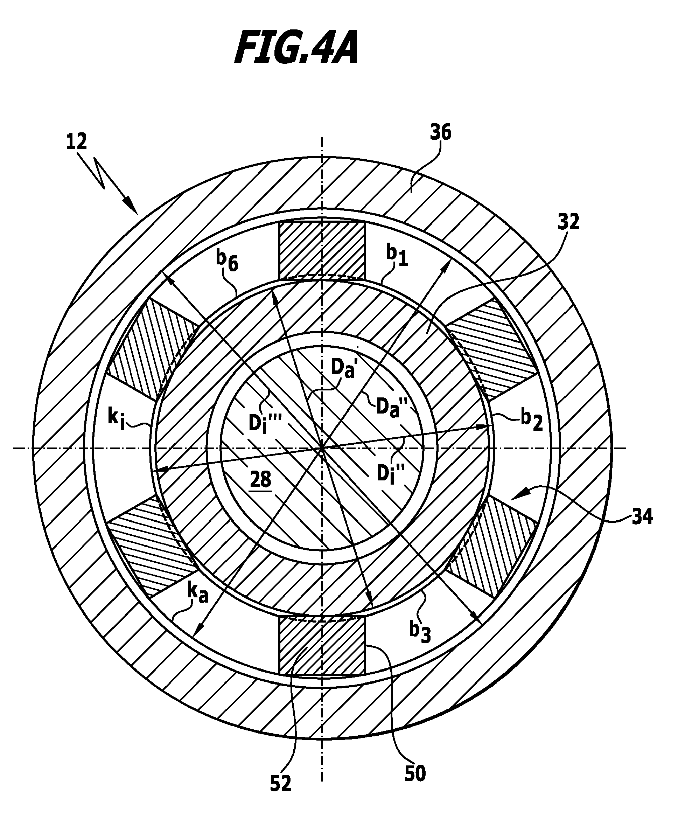

Figur 4A zeigt die Steuerungsvorrichtung im Querschnitt entlang Linie IV - IV in Figur 1.

Die Steuerungsvorrichtung umfasst von außen nach innen betrachtet einen äußeren Schaft 36 und einen inneren Schaft 32, die zwischen sich einen Ringraum bilden in dem das Steuerungselement 34 aufgenommen ist. Im Innern des inneren Schafts 32 ist schließlich als Kopplungselement 28 eine flexible Zug- oder Schubstange angeordnet.

Die sechs Längselemente 52 sind über Schlitze 50 voneinander in Umfangsrichtung getrennt und sind hier nur schematisch gezeigt um das Prinzip der Auslegung der Breite der Längselemente und den Abstand $b_1$, $b_2$ ... $b_6$ zwischen denselben zu verdeutlichen.

In der Figur 4A sind die einzelnen Abmessungen wie folgt bezeichnet:

Da'	= Außendurchmesser des inneren Schafts 32;
Di''	= Durchmesser eines Kreises $K_i$, der durch die radial innen liegenden Kanten der Längselemente 52 verläuft;
Da''	= Durchmesser eines Kreises $K_a$, der durch die radial außen liegenden Kanten der Längselemente 52 verläuft;
Di'''	= Innendurchmesser des äußeren Schafts 36;
$b_{1, 2, ...}$	= Abstand der Längselemente 52 voneinander in Umfangsrichtung entlang des Kreises mit dem Durchmesser Di' gemessen.

Bei den bei den erfindungsgemäßen Steuerungsvorrichtungen typischerweise auftretenden Kräften kann bei den bevorzugt verwendeten Materialien für die Herstellung des Steuerungselements 34, z.B. Nitinol, davon ausgegangen werden, dass die Materialelastizität nicht zu einer Längenänderung der Längselemente 52 führt.

Bei dieser Bedingung gilt dann die folgende Beziehung (1) für die Dimensionierung des Außendurchmessers Da' bezüglich des Innendurchmessers Di'' und der in Umfangsrichtung gemessenen Abständen $b_{1, 2, ...}$ $b_n$ der Längselemente 50 (im Spezialfall der Figur 4A n=6):

$$Da' > Di'' - 1/\pi * \Sigma\ (b_1 + b_2 + ... b_n) \qquad\qquad (1)$$

Für die Durchmesser untereinander wird vorzugsweise folgende Beziehung (2) zusätzlich eingehalten:

$$Di''' - Da' < 1\ \tfrac{1}{2} * (Da'' - Di'') \qquad\qquad (2)$$

Damit sind auch die Fertigungstoleranzen der einzelnen Schäfte und Längselemente berücksichtigt.

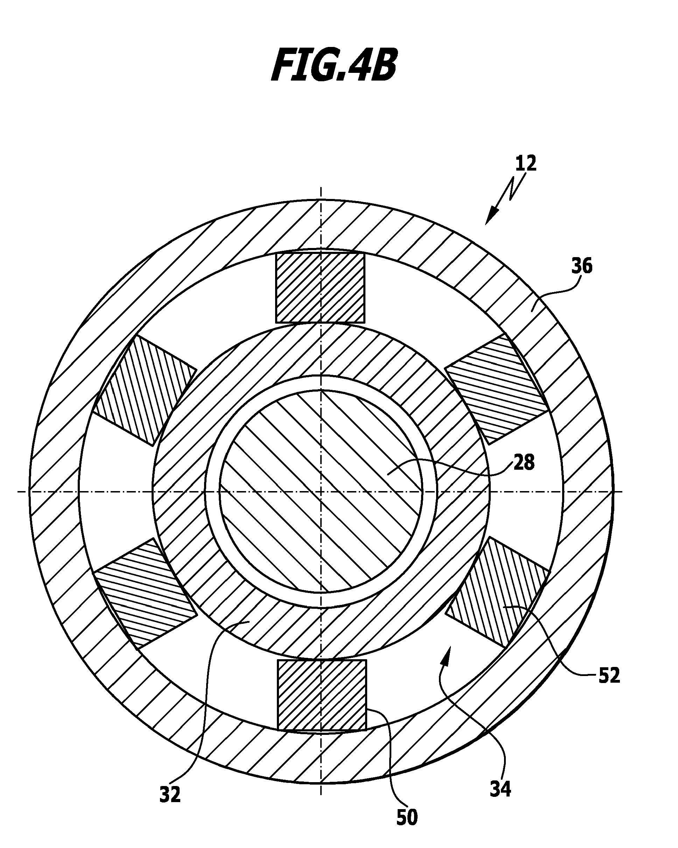

In Figur 4B ist eine Variante der Ausführungsform der Figur 4A gezeigt, bei der die Längselemente 52 an ihren radial innen und außen liegenden Kanten durch den inneren bzw. äußeren Schaft 32, 36 geführt sind.

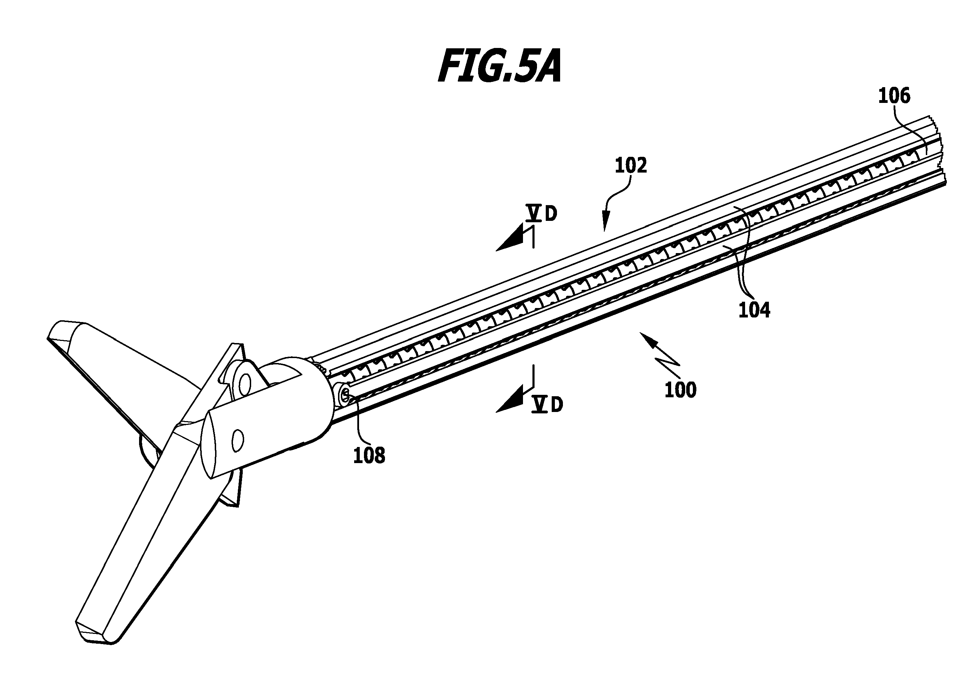

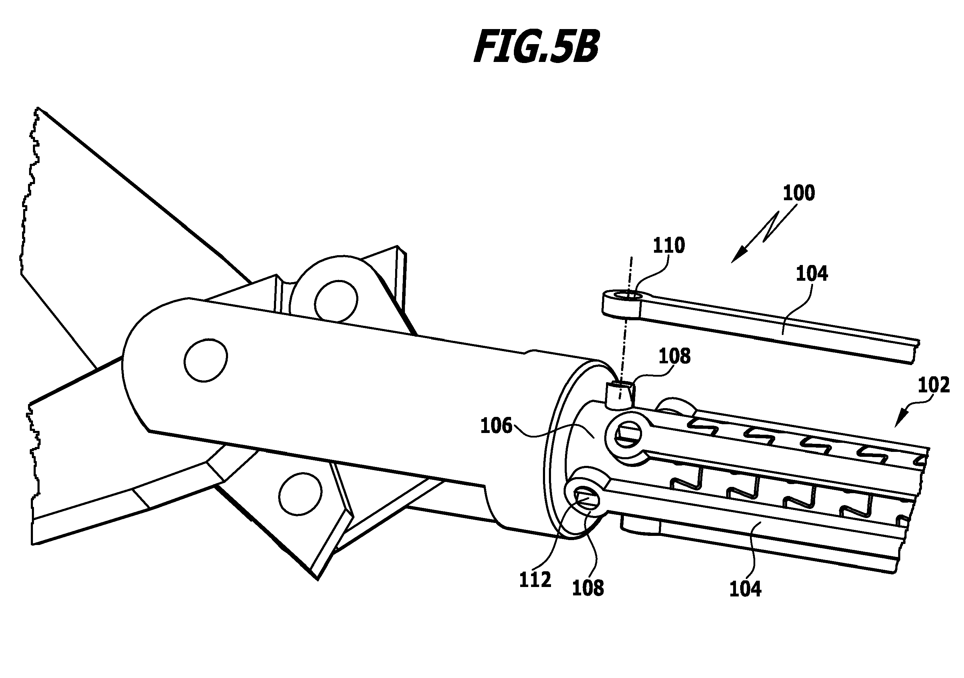

Die Figuren 5A bis 5C zeigen eine alternative Steuerungsvorrichtung 100 mit einem Steuerungselement 102, das anders aufgebaut ist als das Steuerungselement der im Zusammenhang mit den Figuren 1 bis 4 beschriebenen Steuerungsvorrichtung 12. Hier sind an Stelle eines zusammenhängenden Flachmaterials einzelne Stege als Längselemente 104 verarbeitet, die an ihrem proximalen und distalen Ende voneinander beabstandet fixiert sind.

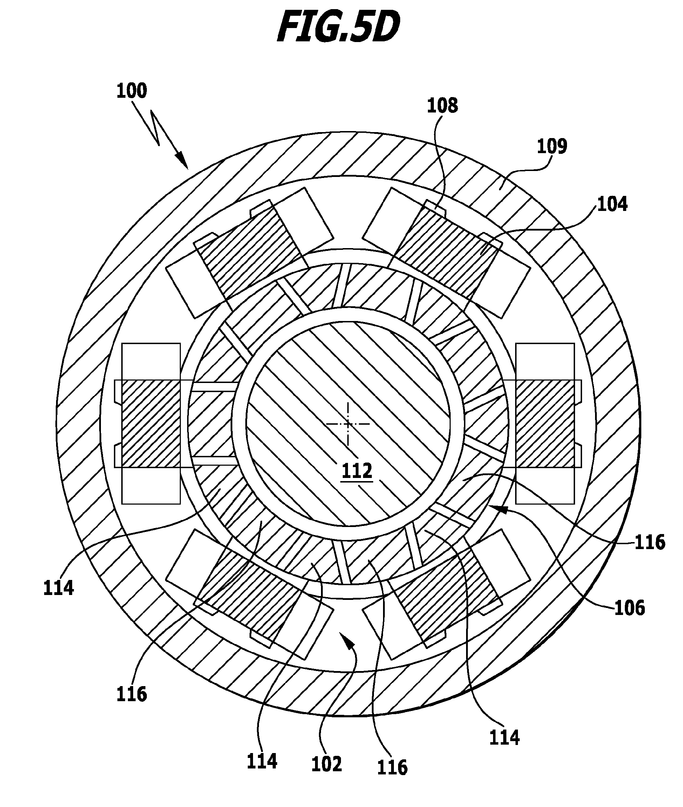

Die Fixierung der Längselemente 104 mit einem ausreichenden Abstand zueinander kann an einem separaten Ringbund geschehen oder diese werden bevorzugt, wie im Rahmen der Figuren 5A bis 5C gezeigt, am proximalen bzw. distalen Ende des inneren Schaftes 106 festgelegt. Der innere Schaft 106 ebenso wie der zugehörige äußere Schaft (vgl. den Schaft 109 in Figur 5D), der hier der Einfachheit halber nicht dargestellt ist, können aufgebaut sein wie dies im Zusammenhang mit dem inneren und dem äußeren Schaft der Steuerungsvorrichtung 12 der Figuren 1 bis 4 beschrieben wurde.

Zur Festlegung der Längselemente 104 am proximalen und distalen Ende des inneren Schafts 106 können an diesem Vorsprünge 108 vorgesehen sein, während die Längselemente 104 an ihrem proximalen und distalen Ende jeweils mit einer Öffnung 110 versehen sind, in die der Vorsprung 108 eingreifen kann.

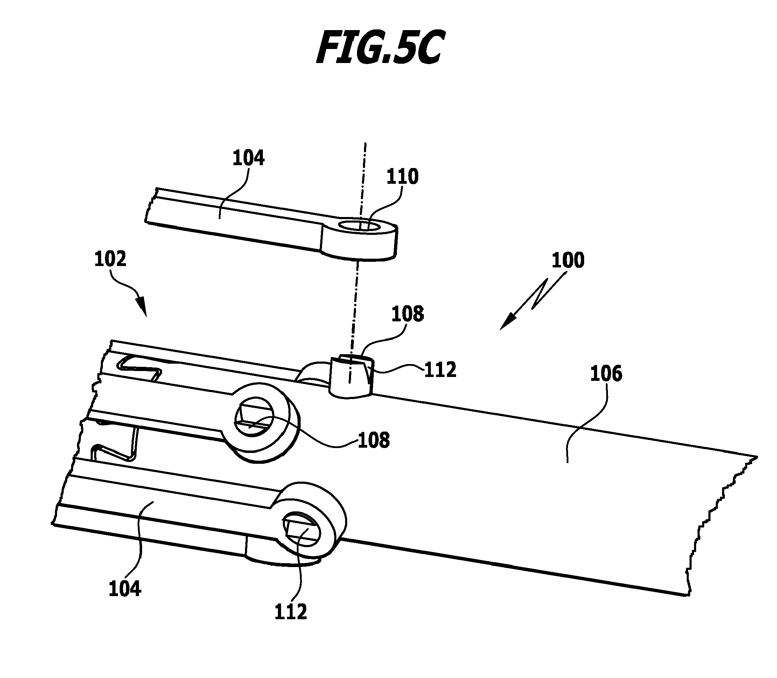

Besonders bevorzugt sind wie in den Figuren 5A und 5C im Einzelnen gezeigt, Vorsprünge 108, die eine keilförmige Nut 112 umfassen, die nach einer Platzierung der Längselemente 104 etwas aufgespreizt werden kann, so dass zusätzlich zu dem Formschluss zwischen Vorsprung 108 und Durchgangsöffnung 110 ein Kraftschluss vorhanden ist, der die Längselemente 104 an dem inneren Schaft 106 für die Zwecke der weiteren Montage der Steuerungsvorrichtung fixiert. Der Kraftschluss braucht nicht besonders stark ausgeprägt sein, so dass die Längselemente 104 insbesondere lösbar an dem inneren Schaft 106 montiert sind, da im vollständig zusammengebauten Zustand der Steuerungsvorrichtung 100 sich über den Bereich der Vorsprünge 108 und den Ösen 110 am jeweiligen Ende der Längselemente 104 ein äußerer Schaft 109 erstreckt, der, wie anhand der Schnittzeichnung in Figur 5D ersichtlich, die Längselemente 104 bzw. deren Durchgangsöffnungen 110 permanent im Eingriff mit den Vorsprüngen 108 hält.

Bei dieser beschriebenen Lösung wird insbesondere bei einer größeren Anzahl von Längselementen 104, wie im vorliegenden Beispiel von sechs oder mehr, die Verbindung zwischen den Längselementen 104 und dem proximalen bzw. distalen Endbereich des inneren Schafts 106 so vorgenommen, dass die Vorsprünge 108 in Längsrichtung, d.h. in Axialrichtung des inneren Schafts 106, etwas versetzt zueinander angeordnet sind, so dass die erweiterten Bereiche der Längselemente 104, die die Öse 110 beinhalten, ohne gegenseitigen Kontakt montierbar sind.

Die Figur 5D zeigt die Steuerungsvorrichtung 100 der Figur 5A im Querschnitt in vollständig zusammengesetzter Form, wobei von außen nach innen gesehen ein äußerer Schaft 109 den inneren Schaft 106 und das aus einzelnen Längselementen 104 gebildete Steuerungselement 102 umgibt und das Steuerungselement in seiner Position fixiert. Im Inneren des inneren Schafts 106 ist noch ein Kopplungselement 112 gezeigt, ähnlich dem Kopplungselement 28 der Ausführungsform der Figuren 1 bis 4. Figur 5D zeigt darüber hinaus noch die Ringelemente 114 und 116, die über Vor- und Rücksprünge gelenkig miteinander verbunden sind. Ein entsprechender Aufbau kann auch beim äußeren Schaft 109 vorhanden sein (Einzelheiten hierzu sind in Figur 5D weggelassen).

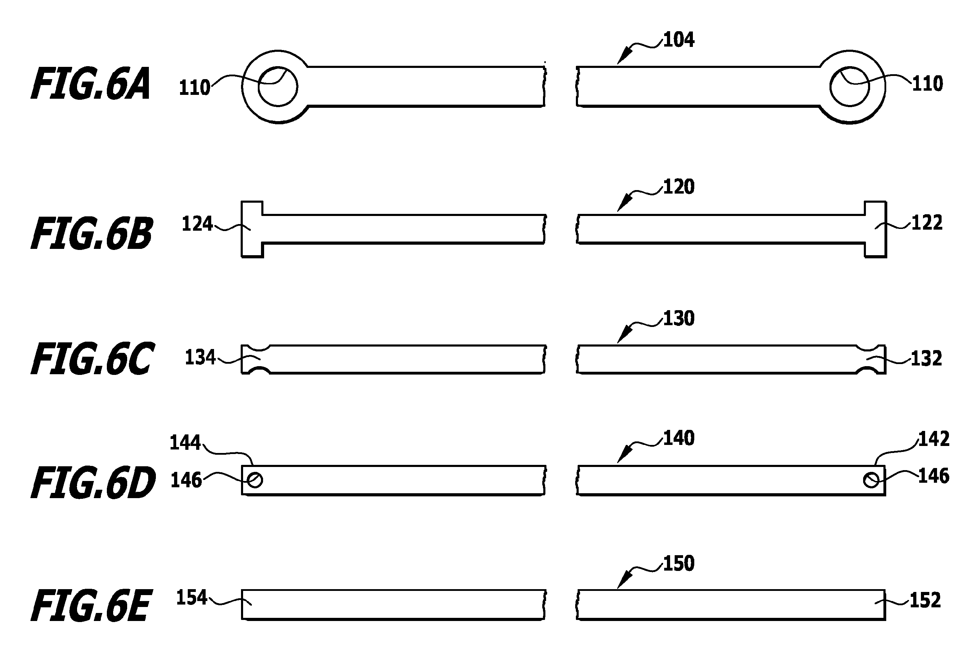

Alternativ zu den in den Figuren 5A bis 5D beschriebenen Längselementen 104 zur Bildung des Steuerelements 102 bieten sich die unterschiedlichsten Varianten an, die beispielhaft in den Figuren 6A bis 6E dargestellt sind, wobei zum besseren Vergleich die Figur 6A nochmals das Längselement 104 darstellt.

Figur 6B zeigt ein alternatives Längselement 120, dessen proximales und distales Ende 122, 124 jeweils T-förmig ausgebildet ist.

Diese T-förmig ausgebildeten proximalen und distalen Enden können in entsprechend ausgebildeten Aufnahmen am inneren Schaft 106 oder einem separaten Ringbund eingelegt werden und stellen so eine Fixierung der Längselemente 120 mit Abstand in Umfangsrichtung gegeneinander her und bieten außerdem eine Verbindung, über die die Kräfte vom proximalen auf das distale Ende Steuerungsvorrichtung 100 übertragbar sind.

Eine weitere Variante ist in der Figur 6C gezeigt, wobei dort ein Längselement 130 mit taillierten proximalen und distalen Endbereichen 132 bzw 134 vorgesehen ist. Diese taillierten Enden 132 und 134 sind mit etwas weniger Platzbedarf an dem proximalen und distalen Ende eines inneren Schafts 106 oder aber auch an einem separaten Ringbund form- und/oder kraftschlüssig fixierbar.

Die Figur 6D zeigt ein Längselement 140 das ähnlich wie das Längselement 104 an seinen proximalen und distalen Enden 142, 144 eine Durchgangsöffnung 146 aufweist, die einen Vorsprung auf Seiten des inneren Schafts 106 bzw. einem separat vorgesehenen Ringbund aufnehmen kann.

Schließlich ist die einfachste Version der separaten Längselemente in Form eines Längselements 150 in Figur 6E gezeigt, das mit seinen proximalen und distalen Enden 152, 154 stoffschlüssig an einem Ringbund und gegebenenfalls auch an einem proximalen und distalen Ende eines inneren Schafts 106 fixierbar ist, beispielsweise durch Kleben oder Schweißen.

Eine stoffschlüssige Verbindung an einem Ringbund oder dem inneren Schaft ist als Alternative auch bei den Längselementen 104, 120, 130, 140 und den Figuren 6A bis 6D denkbar.

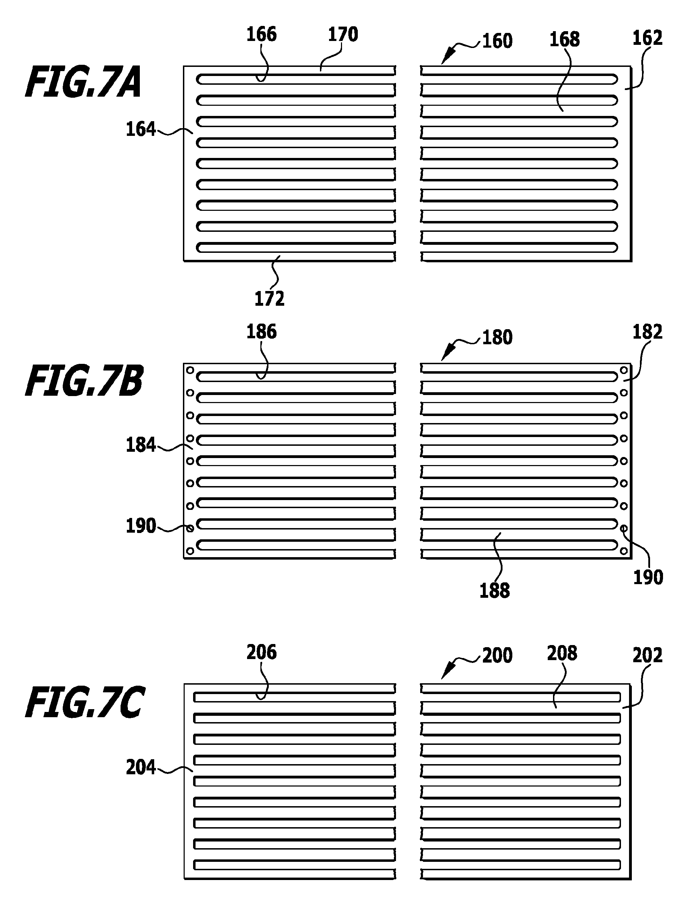

In der Figur 7 sind in der Übersicht verschiedene Flachmaterialen dargestellt, die, zum Röhrchen geformt, ein Steuerungselement, wie es im Rahmen der Steuerungsvorrichtung 12 der Ausführungsform der Figuren 1 bis 4 verwendet wurde, bilden.

Die Figur 7A zeigt ein Flachmaterial 160 mit einem proximalen Ende 162 und einem distalen Ende 164.

Zwischen den proximalen und distalen Enden 162, 164 erstrecken sich im Flachmaterial 160 Schlitze 166, die die mit den proximalen Enden und distalen Enden 162, 164 verbundenen Längselemente 168 voneinander beabstandet halten, wobei die Längselemente 168 mit den proximalen und distalen Enden 162, 164 einstückig ausgebildet sind.

Die Schlitze 166 sind an ihren proximalen und distalen Enden mit einem Radius verrundet ausgebildet, der ungefähr der Hälfte der Breite der Schlitze 166 zwischen benachbarten Längselementen 168 entspricht.

Wenn das Flachmaterial 160 zu einem Röhrchen geformt wird, können die lateral außen liegenden Längselemente 170 und 172 direkt aneinander liegen und können auch mit einer Naht miteinander verbunden werden. In einer so gestalteten Ausführungsform wird die Breite der Längselemente 170, 172 ungefähr die Hälfte der Breite der Längselemente 168 betragen.

Alternativ könnte vorgesehen sein, dass die lateral angeordneten Längselement 170, 172 mit der derselben Breite wie die Längselemente 168 ausgebildet sind, wobei dann die lateralen Längselemente 170, 172 im zum Röhrchen gerollten Zustand des Flachmaterials 160 auf einem Abstand gehalten sind, entsprechend der Breite des Schlitzes 166 im Flachmaterial 160.

Bei einer weiteren Variante kann eines der lateralen Längselemente 170, 172 entfallen, während der proximale und distale Endbereich 162, 164 dieselbe Breite aufweist wie in Figur 7A. Dann ist ein überstehender Bereich über das letzte der Längselemente in Querrichtung vorhanden, der dann mit einer Naht mit dem lateralen Ende des Flachmaterials 160 verbunden werden kann.

Während das Flachmaterial 160 zu einem Steuerungselement in Röhrchenform aufgerollt wird und als solches einfach zwischen einem inneren und einem äußeren Schaft gehalten werden kann und so seiner Bestimmung Kräfte vom proximalen auf das distale Ende einer Steuerungsvorrichtung zu übertragen voll auf genügt, kann zur zusätzlichen Sicherung des Steuerungselements in der zusammengesetzten Steuerungsvorrichtung am proximalen und distalen Ende des Flachmaterials eine Reihe von Durchgangsöffnungen vorgesehen sein, die korrespondierend ausgebildete Vorsprünge am inneren Schaft aufnehmen können, ähnlich wie dies im Zusammenhang mit der Ausführungsform einer Steuerungsvorrichtung 12 im Zusammenhang mit den Figuren 1 bis 4 beschrieben wurde.

Eine solche Weiterbildung zeigt die Figur 7B, bei der ein Flachmaterial 180 mit proximalen und distalen Enden 182, 184, zwischen denen sich wiederum von Schlitzen 186 auf Abstand gehaltene Längselemente 188 erstrecken, analog der Ausführungsform, die im Zusammenhang mit der Figur 7A beschrieben wurde. Im Bereich der proximalen und distalen Enden 182, 184 sind jedoch zusätzlich Durchgangsöffnungen 190 vorgesehen.

Figur 7C zeigt eine weiteres alternatives Flachmaterial 200 mit einem proximalen Ende 202 und einem distalen Ende 204 bei dem sich wiederum Schlitze 206 vom proximalen Ende 202 bis zum distalen Ende 204 erstrecken und Längselemente 208 voneinander auf Abstand gegenseitig fixieren.

Anders als bei der Ausführungsform des Flachmaterials 160 der Figur 7C sind hier die Enden der Schlitze 206 rechteckig ausgebildet.

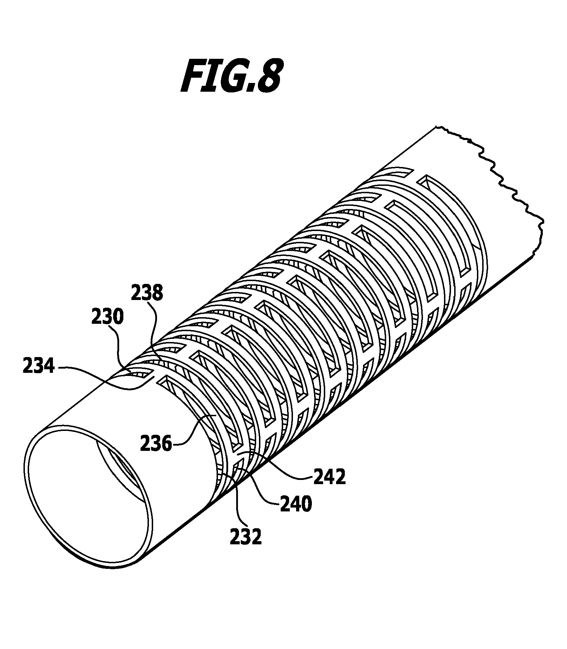

Figur 8 zeigt schließlich noch eine alternative Ausführungsform eines Gelenkabschnitts für einen inneren und/oder äußeren Schaft bei dem die Gelenkfunktion dadurch herstellt wird, dass in Umfangsrichtung verlaufende Schlitze versetzt zueinander angeordnet sind.

Im Fall des Ausführungsbeispiels der Figur 8 sind in Umfangsrichtung jeweils zwei Schlitze 230, 232 vorgesehen, die nur durch einen schmalen Steg 234 voneinander getrennt sind. In Axialrichtung folgt in einem kurzen Abstand 236 wiederum ein Paar Schlitze 238, 240, die in Umfangsrichtung verlaufen und die durch einen schmalen Steg 242 voneinander getrennt sind. Die paarweise Anordnung der Schlitze 230, 232 und 238, 240 erfolgt in Umfangsrichtung um jeweils 90° versetzt, so dass die Gelenkzone Bewegungen in alle Richtungen mit ungefähr dem gleichen Kraftaufwand zulässt.

Das Muster der alternierend angeordneten Schlitze 230, 232 und 238, 240 setzt sich über die gesamte Gelenkzone des Schaftes fort und erlaubt bei einem Material, wie z.B. Nitinol eine sehr einfache, einstückige gelenkige Struktur auszubilden.

## Patentansprüche

1. Steuerungsvorrichtung (12), insbesondere zur Verwendung in Endoskopen oder dergleichen, mit einem proximalen und einem distalen, jeweils eine Gelenkzone umfassenden Endabschnitt (18, 20) sowie einem dazwischen angeordneten Mittelabschnitt,

   mit einem äußeren hohlzylindrischen Schaft (36), einem inneren, vorzugsweise hohlzylindrischen Schaft (32) sowie mit einem zwischen diesen Schäften angeordneten Steuerungselement (34) mit zwei oder mehr sich im Wesentlichen vom proximalen zum distalen Endabschnitt der Steuerungsvorrichtung erstreckenden, Kraft übertragenden Längselementen (52) zur mechanischen Kopplung der Bewegung des distalen Endabschnitts (20) mit der Bewegung des proximalen Gelenkabschnitts (18),

   wobei die Längselemente (52) in Umfangsrichtung der Steuerungsvorrichtung (12) in im Wesentlichen regelmäßigen Winkelabständen $b_1$, $b_2$, ... $b_n$ voneinander beabstandet angeordnet und im Bereich ihrer proximalen und distalen Enden jeweils in Umfangsrichtung gegeneinander fixiert sind, **dadurch gekennzeichnet, dass** der Querschnitt der Längselemente (52) im Wesentlichen rechteckförmig ausgebildet ist und dass die Winkelabstände $b_1$, $b_2$, ... $b_n$ der Längselemente (52) zueinander, in Umfangsrichtung gemessen, so gewählt sind, dass die Kraft übertragenden Längsele-mente (52) in Umfangsrichtung berührungsfrei und gegebenenfalls in Radialrichtung an dem äußeren und/oder dem inneren Schaft (36;32) anliegend geführt sind.

2. Steuerungsvorrichtung (12) nach Anspruch 1, **dadurch gekennzeichnet, dass** die Längselemente (52) in Radialrichtung an dem äußeren und dem inneren Schaft (36;32) anliegen und geführt sind.

3. Steuerungsvorrichtung (12) nach Anspruch 1 oder 2, **dadurch gekennzeichnet, dass** das Steuerungselement (12) mit seinen Längselementen (52), von einem längs geschlitzten, zum Rohr geformten Flachmaterial oder von einzelnen stabförmigen Elementen, welche an ihrem proximalen und distalen Ende, vorzugsweise über einen Ringbund, in Umfangsrichtung gegeneinander fixiert sind, gebildet ist.

4. Steuerungsvorrichtung (12) nach Anspruch 1 bis 2, **dadurch gekennzeichnet, dass** der Außendurchmesser des äußeren Schafts (36) im Bereich von ca. 1 mm bis ca. 20 mm und der Innendurchmesser des inneren Schafts (32) im Bereich von ca. 0,05 mm bis ca. 5 mm gewählt wird.

5. Steuerungsvorrichtung (12) nach einem der Ansprüche 1 bis 4, **dadurch gekennzeichnet, dass** die distalen Enden der Längselemente (52) in Umfangsrichtung in Winkelpositionen festgelegt sind, die von den Winkelpositionen, in denen die jeweils zugehörenden proximalen Enden festgelegt sind, verschieden sind, wobei die Winkelpositionen vorzugsweise in Umfangsrichtung um ca. 10 ° bis 360 °, insbesondere um ca. 45 ° bis 315 ° differieren.

6. Steuerungsvorrichtung (12) nach einem der Ansprüche 1 bis 5, **dadurch gekennzeichnet, dass** die Kraft übertragenden Längselemente (52) mindestens über einen Teil ihrer Länge schraubenlinienförmig zwischen den Schäften angeordnet sind (36;32), wobei optional die Kraft übertragenden Längselemente (52) im Bereich der proximalen und/oder distalen Enden im Wesentlichen parallel zur Längsrichtung der Steuervorrichtung (12) und in einem dazwischen liegenden Bereich schraubenlinienförmig angeordnet sind.

7. Steuerungsvorrichtung (12) nach Anspruch 6, **dadurch gekennzeichnet, dass** die Kraft übertragenden Längselemente (52) einen oder mehr Abschnitte im Bereich zwischen ihren proximalen und distalen Enden aufweisen, welche parallel zur Längsrichtung der Steuerungsvorrichtung (12) angeordnet sind.

8. Steuerungsvorrichtung (12) nach einem der Ansprüche 1 bis 7, **dadurch gekennzeichnet, dass** die Gelenkabschnitte (40,44;38,42) des inneren und/oder äußeren Schafts (32;36) eine Mehrzahl an Ringsegmenten umfassen, welche in Axialrichtung jeweils einen ersten und einen zweiten Endbereich aufweisen, wobei der erste Endbereich zwei oder mehr in Axialrichtung abstehende Vorsprünge und der zweite Endbereich zwei oder mehr die Vorsprünge aufnehmende Rücksprünge umfasst, wobei die Vor- und Rücksprünge in regelmäßigen Abständen in Umfangsrichtung am jeweiligen Ringsegment angeordnet sind, wobei die Ringsegmente über die Vor- und Rücksprünge gelenkig ineinander greifen und wobei die Ringsegmente über die Vor- und Rücksprünge in Axial- und/oder Radialrichtung miteinander formschlüssig verbunden sind.

9. Steuerungsvorrichtung (12) nach Anspruch 8, **dadurch gekennzeichnet, dass** die Vorsprünge an ihrer radial außen liegenden Seite in Umfangsrichtung eine größere Ausdehnung aufweisen als auf ihrer radial innen liegenden Seite und dass die Rücksprünge auf der radial innen liegenden Seite in Umfangsrichtung eine Ausdehnung aufweisen, die kleiner ist als die entsprechende radial außen liegende Ausdehnung des in den Rücksprung eingreifenden Vorsprungs.

10. Steuerungsvorrichtung (12) nach Anspruch 8 oder 9, **dadurch gekennzeichnet, dass** die Vorsprünge an ihrem dem Ringsegment abgewandten freien Ende in Umfangsrichtung eine größere Ausdehnung aufweisen als benachbart zum Ringsegment und dass die Rücksprünge an ihrem offenen Ende eine kleinere Ausdehnung in Umfangsrichtung aufweisen als die Ausdehnung des in den Rücksprung eingreifenden Vorsprungs an seinem freien Ende.

11. Steuerungsvorrichtung (12) nach einem der Ansprüche 8 bis 10,
**dadurch gekennzeichnet, dass** die Vor- und die Rücksprünge in Umfangs- und oder Radialrichtung mit einem im Wesentlichen trapezförmigen Querschnitt ausgebildet sind.

12. Steuerungsvorrichtung (12) nach einem der Ansprüche 1 bis 7, **dadurch gekennzeichnet, dass** die Gelenkzone(n) des äußeren und/oder inneren Schafts (36;32) einen Wandabschnitt umfassen, in dem mehrere voneinander beabstandete, in Umfangsrichtung verlaufende Schlitze angeordnet sind, welche bevorzugt als die Zylinderwand des Schafts vollständig durchdringende Schlitze ausgebildet sind, wobei vorzugsweise in Umfangsrichtung zwei oder mehr, insbesondere drei oder mehr Schlitze hintereinander angeordnet sind, wobei optional in Axialrichtung drei oder mehr Schlitze nebeneinander und optional gegeneinander versetzt angeordnet sind.

13. Steuerungsvorrichtung (12) nach einem der Ansprüche 1 bis 12,
**dadurch gekennzeichnet, dass** mindestens eine der Gelenkzonen elastisch ausgebildet ist.

14. Steuerungsvorrichtung (12) nach einem der Ansprüche 1 bis 13,
**dadurch gekennzeichnet, dass** die Längselemente (52) des Steuerungselements (12) aus einer Stahllegierung oder Nitinol hergestellt sind.

15. Steuerungsvorrichtung (12) nach einem der Ansprüche 1 bis 14,
**dadurch gekennzeichnet, dass** mindestens einer der äußeren und inneren Schäfte (36;32) einen zwischen den proximalen und distalen Gelenkzonen angeordneten biegesteifen Abschnitt aufweist.

**FIG.1**

FIG.2

FIG.2A

# FIG.3A

EP 3 138 466 A1

FIG.3B

# FIG.4A

# FIG.4B

**FIG.5A**

EP 3 138 466 A1

FIG.5B

EP 3 138 466 A1

# FIG.5C

EP 3 138 466 A1

# FIG.5D

**FIG.6A** 104 110 110

**FIG.6B** 120 124 122

**FIG.6C** 130 134 132

**FIG.6D** 140 144 146 142 146

**FIG.6E** 150 154 152

**FIG.7A**

**FIG.7B**

**FIG.7C**

# FIG.8

**Europäisches Patentamt**
**European Patent Office**
**Office européen des brevets**

# EUROPÄISCHER RECHERCHENBERICHT

**Nummer der Anmeldung**

EP 16 19 1510

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | WO 2009/112060 A1 (FORTIMEDIX B V [NL]; VERBEEK MARCEL ANTONIUS ELISAB [NL]) 17. September 2009 (2009-09-17) * Seite 4, Zeile 4 - Seite 5, Zeile 24; Abbildungen 1,2,4,7,9,19-23 * ----- | 1-15 | INV. A61B1/005 G02B23/24 A61B17/295 A61M25/01 A61B1/00 |
| Y | US 2009/299343 A1 (ROGERS THEODORE W [US]) 3. Dezember 2009 (2009-12-03) * Absätze [0030], [0034], [0035]; Abbildungen 1,2,4A-F * ----- | 1-15 | |
| Y | DE 20 2009 012698 U1 (AESCULAP WERKE AG [DE]) 12. Mai 2010 (2010-05-12) * das ganze Dokument * ----- | 4-15 | |
| A | DE 20 2009 012795 U1 (AESCULAP WERKE AG [DE]) 21. Januar 2010 (2010-01-21) * das ganze Dokument * ----- | 1-15 | |
| A | US 2011/004157 A1 (DEWAELE FRANK [BE] ET AL) 6. Januar 2011 (2011-01-06) * das ganze Dokument * ----- | 1-15 | RECHERCHIERTE SACHGEBIETE (IPC) A61B G02B A61M |

Der vorliegende Recherchenbericht wurde für alle Patentansprüche erstellt

| Recherchenort | Abschlußdatum der Recherche | Prüfer |
|---|---|---|
| München | 13. Januar 2017 | Schindler, Martin |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 16 19 1510

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-01-2017

| Im Recherchenbericht angeführtes Patentdokument | Datum der Veröffentlichung | Mitglied(er) der Patentfamilie | Datum der Veröffentlichung |
|---|---|---|---|
| WO 2009112060 A1 | 17-09-2009 | BR PI0822419 A2 | 16-06-2015 |
| | | CN 102006815 A | 06-04-2011 |
| | | CN 104337491 A | 11-02-2015 |
| | | EA 201071049 A1 | 29-04-2011 |
| | | EA 201300520 A1 | 30-12-2013 |
| | | EP 2273911 A1 | 19-01-2011 |
| | | EP 2762058 A1 | 06-08-2014 |
| | | EP 2915476 A1 | 09-09-2015 |
| | | ES 2541117 T3 | 16-07-2015 |
| | | ES 2562918 T3 | 09-03-2016 |
| | | JP 5331826 B2 | 30-10-2013 |
| | | JP 2011517290 A | 02-06-2011 |
| | | KR 20110002030 A | 06-01-2011 |
| | | US 2011034764 A1 | 10-02-2011 |
| | | US 2015151080 A1 | 04-06-2015 |
| | | WO 2009112060 A1 | 17-09-2009 |
| US 2009299343 A1 | 03-12-2009 | US 2009299343 A1 | 03-12-2009 |
| | | US 2014005639 A1 | 02-01-2014 |
| | | US 2015025320 A1 | 22-01-2015 |
| DE 202009012698 U1 | 12-05-2010 | DE 102009042488 A1 | 02-12-2010 |
| | | DE 202009012698 U1 | 12-05-2010 |
| | | EP 2434939 A1 | 04-04-2012 |
| | | JP 5624610 B2 | 12-11-2014 |
| | | JP 2012527917 A | 12-11-2012 |
| | | US 2012116163 A1 | 10-05-2012 |
| | | WO 2010136272 A1 | 02-12-2010 |
| DE 202009012795 U1 | 21-01-2010 | CN 102448359 A | 09-05-2012 |
| | | DE 102009042490 A1 | 09-12-2010 |
| | | DE 202009012795 U1 | 21-01-2010 |
| | | EP 2434940 A1 | 04-04-2012 |
| | | JP 2012527919 A | 12-11-2012 |
| | | RU 2011153010 A | 10-07-2013 |
| | | US 2012130173 A1 | 24-05-2012 |
| | | WO 2010136275 A1 | 02-12-2010 |
| US 2011004157 A1 | 06-01-2011 | CN 101938933 A | 05-01-2011 |
| | | CN 103654694 A | 26-03-2014 |
| | | EP 2259710 A2 | 15-12-2010 |
| | | EP 2664270 A1 | 20-11-2013 |
| | | JP 5409655 B2 | 05-02-2014 |
| | | JP 2011510773 A | 07-04-2011 |
| | | US 2011004157 A1 | 06-01-2011 |
| | | US 2013253481 A1 | 26-09-2013 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT**
**ÜBER DIE EUROPÄISCHE PATENTANMELDUNG NR.**

EP 16 19 1510

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-01-2017

| Im Recherchenbericht angeführtes Patentdokument | Datum der Veröffentlichung | Mitglied(er) der Patentfamilie | Datum der Veröffentlichung |
|---|---|---|---|
| | | WO    2009098244 A2 | 13-08-2009 |

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2005067785 A1
- WO 2009098244 A2
- WO 2009112060 A
- US 5741429 A
- WO 2009112060 A1